Europäisches Patentamt

European Patent Office

Office européen des brevets

(19)

(11)  EP 1 138 762 A1

## (12) EUROPEAN PATENT APPLICATION
published in accordance with Art. 158(3) EPC

(43) Date of publication:
04.10.2001 Bulletin 2001/40

(51) Int Cl.7: **C12N 15/00**, C12Q 1/68,
G01N 33/50

(21) Application number: 99959692.7

(22) Date of filing: 08.12.1999

(86) International application number:
PCT/JP99/06867

(87) International publication number:
WO 00/34457 (15.06.2000 Gazette 2000/24)

(84) Designated Contracting States:
AT BE CH CY DE DK ES FI FR GB GR IE IT LI LU
MC NL PT SE
Designated Extension States:
AL LT LV MK RO SI

(30) Priority: 10.12.1998 JP 35127698

(71) Applicant: Takara Shuzo Co, Ltd.
Kyoto-shi, Kyoto 612-8061 (JP)

(72) Inventors:
• UEDA, Minoru, 412, Hamoparesu-Kusatsu
Kusatsu-shi, Shiga 525-0025 (JP)
• OKAMOTO, Sachiko
Joyo-shi, Kyoto 610-0121 (JP)

• OZAKI, Aya
Otsu-shi, Shiga 520-0037 (JP)
• MINENO, Junichi
Uji-shi, Kyoto 611-0002 (JP)
• KIMIZUKA, Fusao
Omihachiman-shi, Shiga 523-0056 (JP)
• ASADA, Kiyozo
Koka-gun, Shiga 520-3333 (JP)
• KATO, Ikunoshin
Uji-shi, Kyoto 611-0028 (JP)

(74) Representative: Grund, Martin, Dr. et al
Dr. Volker Vossius,
Patentanwaltskanzlei,
Holbeinstrasse 5
81679 München (DE)

## (54) METHOD FOR IMMOBILIZING OLIGONUCLEOTIDE ON A CARRIER

(57)   A method for immobilizing an oligonucleotide on a carrier by spotting a buffer containing the oligonucleotide onto the carrier, characterized in that the oligonucleotide is immobilized on the carrier via a covalent bond.

**Description**

Technical Field

The present invention relates to a method for immobilizing an oligonucleotide onto a solid support, which is useful for the preparation of a DNA microarray and the like. The present invention also relates to a material onto which an oligonucleotide is immobilized according to said method and a method for detecting a target nucleic acid using said material.

Background Art

With the progress of projects for analyzing genomes of organisms including human, the structures of genomes of various organisms have gradually become elucidated. In parallel, techniques for analyzing the functions of the genomes have been developed in order to utilize the vast information on the genomes for the development of pharmaceuticals or the maintenance or improvement of health. DNA chip technology is one of the principal techniques noticeable for this purpose. A DNA chip is a microarray (DNA array) in which a number of various genes or DNA fragments are arrayed and immobilized onto a solid support such as a slide glass. The DNA chip is very useful as means to greatly accelerate the analysis of expression, mutation or polymorphism of a gene.

It is necessary to immobilize a DNA onto a support in order to prepare a DNA chip. Known methods for immobilizing a DNA is generally classified into two groups.

One is a group of methods in which a DNA is chemically synthesized on a tip of a linker covalently bonded to a support, for example, as described in Science, 251:767-773 (1991) and Nucleic Acids Research, 20:1679-1684 (1992). Such a method can be used to immobilize a DNA (an oligonucleotide) which can be synthesized and of which the sequence is known. It is said that the yield in each step of this method is lower than that of conventional solid phase synthesis. Furthermore, the method requires a micro apparatus for completely sealing reaction sites.

Another is a group of methods in which a DNA which has been synthesized beforehand or a DNA which has been prepared by polymerase chain reaction (PCR) (a PCR amplification product) is covalently (e.g., Nucleic Acids Research, 22:5456-5465 (1994)) or non-covalently (or electrostatically; e.g., Science, 270:467-470 (1995)) bonded to a support. It is supposed that one can immobilize any DNAs according to such a method.

However, for example, in case of non-covalent bonding, it is difficult to immobilize a short chain DNA (an oligonucleotide) such as a synthetic DNA. If a long chain DNA such as a PCR product is to be immobilized onto a support via a non-covalent bond, the DNA is generally immobilized by denaturing the DNA dissolved in a salt solution such as sodium chloride/sodium citrate (SSC), tris-hydrochloride/EDTA (TE) or phosphate buffered saline (PBS), and then spotting it onto a slide glass (a support) coated with a polycation such as polylysine or polyethylenimine. In this case, it is known that a considerable amount of the DNA is detached from the support during the subsequent step of washing or hybridization with a target nucleic acid. This phenomenon is a factor that reduces the sensitivity of detecting a target nucleic acid. Furthermore, the above-mentioned method as described in Nucleic Acids Research, 22: 5456-5465 (1994) is not convenient because it requires treating the support in a nonaqueous system.

It is necessary to heat- or alkali-denature a DNA beforehand in order to increase the immobilization efficiency to a support. It is known that the immobilization efficiency is remarkably reduced in the absence of denaturing. In addition, if a number of DNA solutions are to be immobilized onto a slide glass using an instrument for preparing DNA chips (an arrayer), the denaturing step make the entire steps complicated, making it difficult to automate the procedure.

The absolute quantity of an immobilized DNA is increased by increasing the concentration of the DNA in a solution to be spotted onto a support for immobilizing the DNA. However, as a result, the immobilization rate to the support is decreased and the loss of the DNA used is increased.

An instrument for preparing DNA chips (an arrayer) is used when a high-density DNA array in which dots for various DNAs are formed in a unit area is prepared. In case of the preparation of a high-density DNA array, the volume of a DNA solution to be spotted onto a support using a certain type of instrument may be quite small. Thus, the DNA solution may be dried before it diffuses on the surface of the support, resulting in formation of ununiform dots.

If a target nucleic acid is detected using a DNA array, the detection sensitivity depends on the amount of immobilized DNA in a unit area (or in a dot) . Thus, the way of increasing the amount of immobilized DNA in a dot, in other words, the way of increasing the density of an immobilized DNA, has become a very important problem. Furthermore, the way of spotting the DNA solution while allowing it to diffuse uniformly has also become very important.

Objects of Invention

The main object of the present invention is to provide (1) an effective method for immobilizing an oligonucleotide onto a solid support, (2) a material onto which an oligonucleotide is immobilized prepared according to said method, and (3) a method for detecting a target nucleic acid using said material.

Summary of Invention

The present inventors have studied intensively and found that a target nucleic acid of interest can be detected using a material onto which an oligonucleotide is immobilized as a micro dot by spotting a small volume of a solution containing an oligonucleotide at a specific concentration or more onto a support for immobilization. The present inventors have also found that a material onto which an oligonucleotide is stably immobilized can be obtained because the oligonucleotide immobilized according to the above-mentioned method is not readily detached from a support. The present inventors have further found that a target nucleic acid can be detected with high sensitivity by using the material. Thus, the present invention has been completed.

Accordingly, the present invention provides the following:

(1) a method for immobilizing an oligonucleotide onto a support, the method comprising spotting a buffer containing an oligonucleotide onto a support, and immobilizing the oligonucleotide onto the support via a covalent bond;
(2) the method according to (1) above, wherein a functional group is introduced into the oligonucleotide;
(3) the method according to (2) above, wherein the functional groups is introduced at a terminus of the oligonucleotide;
(4) the method according to (2) or (3) above, wherein an amino group is introduced into the oligonucleotide;
(5) the method according to any one of (1) to (4) above, wherein the support has a functional group;
(6) the method according to (5) above, wherein the support has an aldehyde group;
(7) the method according to any one of (1) to (6) above, wherein the oligonucleotide is immobilized onto the support through a spacer between the oligonucleotide and the surface of the support;
(8) the method according to any one of (1) to (7) above, wherein 200 nl or less of the buffer containing the oligonucleotide at a concentration of 1 μM or more is spotted onto the support;
(9) the method according to any one of (1) to (8) above, wherein the support is made from glass or quartz, or is a material prepared by treating the surface of glass or quartz;
(10) the method according to any one of (1) to (9) above, wherein the buffer contains at least one substance selected from the group consisting of morpholine, a morpholine derivative, a salt thereof and a carbonate;
(11) the method according to (10) above, wherein the concentration of at least one substance selected from the group consisting of morpholine, a morpholine derivative, a salt thereof and a carbonate in the buffer is 10 to 500 mM;
(12) a material onto which an oligonucleotide is immobilized, which is prepared according to the method defined by any one of (1) to (11) above;
(13) the material according to (12) above, wherein the oligonucleotide is immobilized at 1.25 fmole/dot or more;
(14) a method for detecting a target nucleic acid, the method comprising detecting a target nucleic acid by using the material onto which an oligonucleotide is immobilized defined by (12) or (13) above; and
(15) the method according to (14) above, comprising hybridizing the material onto which an oligonucleotide is immobilized with the target nucleic acid under stringent conditions.

Detailed Description of the Invention

As used herein, an oligonucleotide refers to a relatively short nucleic acid. Although it is not intended to limit the present invention, it means a nucleic acid of 6 to 100 bases in length. Preferably, a nucleic acid of 8 to 50 bases in length is used in the present invention. The oligonucleotides of the present invention include a DNA, an RNA, a chimeric molecule thereof and a derivative thereof. Such an oligonucleotide can be prepared by chemical or enzymatic synthesis. Alternatively, it can be prepared by chemically or enzymatically treating a naturally occurring nucleic acid. The oligonucleotide of the present invention may be single-stranded or doublestranded. If it is intended to use the oligonucleotide for hybridization, a single-stranded oligonucleotide is usually used.

Examples of the derivatives of the oligonucleotides include one containing in the molecule a nucleotide such as inosine or 7-deazaguanosine which is different from those constituting a normal nucleic acid, one in which a phosphate bond is replaced by a phosphorothioate bond, and one modified for immobilization onto the surface of a support. The modification is not specifically limited. An oligonucleotide into which a functional group such as an amino group, a carboxyl group, a thiol group, an aldehyde group or an epoxy is introduced is exemplified. An oligonucleotide into which the functional group is introduced at the terminus of the oligonucleotide is preferably used in the present invention. Use of an oligonucleotide into which an amino group is introduced is particularly preferable.

A support used for immobilizing an oligonucleotide in the present invention is not limited to specific one as long as it is made from a substantially insoluble material. Examples of the materials include paper, nylon, cellulose, cellulose derivatives such as nitrocellulose, plastics and plastic derivatives such as polycarbonate, polystyrene and polypropylene, magnetic or non-magnetic metals, quartz and glass. The materials may be porous, smooth-surfaced, polymeric or non-polymeric.

The supports to be used in the present invention are not limited to those made from such materials. Any supports which can be used as carries for DNA chips or biosensors can be used as long as they can retain oligonucleotides to be immobilized on their surfaces. For example, a support having a hydrophilic or hydrophobic functional group such as a hydroxyl group, an amine group, a thiol group, an aldehyde group, a carboxyl group, an epoxy group or an acyl group on its surface can be preferably utilized. Use of a support having an aldehyde group is particularly preferable. Such a functional group can be utilized as it is or as an appropriate derivative. For example, in case of a carboxyl group, it can be used as an activated ester derivative such as an N-hydroxysuccinimide ester.

The supports include those having the functional groups on their surfaces as the properties of the materials for the supports. A support that does not have a hydrophilic or hydrophobic functional group on the surface of the support can be used without limitation if it can be made to have a hydrophilic or hydrophobic functional group on its surface by treating the surface.

Examples of such supports prepared by surface treatment include those prepared by treating glass or quartz with a commercially available silane coupling agent such as aminoalkylsilane or with a polycation such as polylysine or polyethylenimine, and those prepared by further treating the thus treated support with glutaraldehyde, phenyldiisocyanate or the like. A slide glass having a functional group as described above being introduced is commercially available from Sigma.

The oligonucleotide can be immobilized through a spacer between the oligonucleotide and the support such that the immobilized oligonucleotide efficiently hybridizes with a target nucleic acid. The spacer is not limited to specific one as long as it provides a space to a certain degree between the oligonucleotide and the support. For example, a nucleic acid (DNA, RNA) that is not complementary to the target nucleic acid or an alkyl chain can be used as a spacer. If an alkyl chain is used as a spacer, an alkyl chain having 3 or more carbons, preferably 6 or more carbons is used.

The method for introducing the spacer is not specifically limited. A method in which an oligonucleotide having a spacer being introduced is used for immobilization to a support, a method in which an oligonucleotide is immobilized after introducing a spacer onto a support, and a method in which an oligonucleotide is immobilized onto a support using a coupling reagent having a spacer can be used.

The method for immobilizing an oligonucleotide onto a support of the present invention is described in detail below.

An oligonucleotide to be immobilized is dissolved in an appropriate buffer to prepare an oligonucleotide solution. The buffer may optionally contain a neutral salt and/or a surfactant.

The buffer used in the present invention is not limited to specific one. Various buffers usually used for dissolving nucleic acids can be used. Sodium chloride/sodium citrate (SSC) is generally used as a buffer. For example, a buffer containing morpholine, a morpholine derivative, a salt thereof or a carbonate as a buffering component can be preferably used.

Examples of morpholine, morpholine derivatives and salts thereof include, but are not limited to, morpholine, a N-alkylmorpholine substituted with a C1-3 alkyl group (e.g., N-methylmorpholine, N-ethylmorpholine or N-propylmorpholine), as well as a salt thereof with a mineral acid (e.g., hydrochloric acid or carbonic acid), an organic acid (e.g., acetic acid, lactic acid or citric acid) or a fatty acid (e.g., lauric acid or stearic acid).

Carbonates include, but are not limited to, a salt of carbonic acid with an alkaline metal, an alkaline earth metal, ammonium or an organic amine such as sodium carbonate, sodium hydrogencarbonate, potassium carbonate, potassium hydrogencarbonate, magnesium carbonate, ammonium carbonate or triethylamine carbonate.

Such an essential buffering component can be used alone, or two or more components can be used in combination.

The buffer for immobilization may further contain other salts such as sodium chloride in addition to the above-mentioned essential buffering component. The buffer may contain a surfactant. The surfactant to be used is not limited to specific one. Any surfactants can be preferably used as long as the homogeneous diffusion of the oligonucleotide solution on the surface of the support is regulated by the addition thereof.

The pH of the buffer for immobilization used in the present invention is adjusted to preferably 7 to 11, more preferably 8 to 10. The salt concentration of the buffer is preferably 10 to 500 mM, more preferably 50 to 200 mM.

Increase in a DNA immobilization rate contributes to increase in sensitivity of detecting a target nucleic acid. Thus, it is preferable to use a buffer containing morpholine, a morpholine derivative, a salt thereof or a carbonate, which results in a high immobilization efficiency, as a buffering component.

It is desirable to make the number of oligonucleotides to be immobilized in a unit area on a support as many as possible in order to allow a number of oligonucleotides to hybridize with a target nucleic acid simultaneously. For this purpose, the volume of an oligonucleotide solution to be spotted is limited to reduce the area of a region (dot) onto which an oligonucleotide is immobilized as much as possible. The signal from a target nucleic acid being hybridized with an immobilized oligonucleotide depends on the amount of the oligonucleotide immobilized in a dot. Thus, it is necessary to immobilize a sufficient amount of an oligonucleotide in a small dot in order to efficiently detect a hybridization signal.

In general, when the concentration of an oligonucleotide contained in a solution used for immobilization to a support is increased, the amount of the immobilized oligonucleotide is increased. However, the amount of oligonucleotide which can be usually used is restricted.

In one embodiment, the method for immobilizing an oligonucleotide of the present invention is characterized in that 200 nl or less of a buffer containing an oligonucleotide at a concentration of 1 μM or more is spotted onto a support. A target nucleic acid being hybridized with an oligonucleotide among a number of oligonucleotides can be detected with high sensitivity by using a support onto which an oligonucleotide is immobilized prepared as described above.

The volume of an oligonucleotide solution to be spotted is further made small if an oligonucleotide array is prepared by forming dots at high density using an instrument for preparing DNA chips or the like for immobilization. For example, the volume of an oligonucleotide solution spotted onto a support using GMS 417 Arrayer from Genetic Microsystems (GMS) is 1 nl or less. In this case, a hybridized target nucleic acid can be detected with sufficient sensitivity if a solution containing an oligonucleotide at a concentration of 100 μM or more is spotted.

An oligonucleotide to be immobilized onto a support is dissolved in a buffer at a concentration suitable for the type of the support or the purpose for which the material having the immobilized oligonucleotide is used. A predetermined volume of the resulting solution is spotted onto a support (e.g., a slide glass) using a micropipette, a microdispenser or an instrument for preparing DNA chips. The support onto which the oligonucleotide has been spotted is incubated, for example, for 1 hour in a humidified incubator. The oligonucleotide is then crosslinked by ultraviolet irradiation. The support is washed in a 0.2% sodium dodecyl sulfate (SDS) aqueous solution followed by distilled water and dried.

A DNA chip onto which oligonucleotide are arrayed and immobilized at high density (an oligonucleotide chip) can be prepared by spotting oligonucleotide solutions onto a support using an instrument for preparing DNA chips (an arrayer). A commercially available instrument can be utilized as an arrayer as long as it can reproducibly prepare a uniform high-density array in a short time. For example, GMS 417 Arrayer can be used. The volume of an oligonucleotide solution spotted using this instrument is usually less than 1 nl. The distance between dots is usually about 300 μm. Using an arrayer with such specification, 5000 or less oligonucleotides can be readily arrayed on a slide glass.

An oligonucleotide can be attached to a support through an amino group in the base moiety of the oligonucleotide. An oligonucleotide modified for immobilization to a support can be attached to the support in a manner suitable for the type of the modification.

For example, an oligonucleotide having an amino group being introduced can be immobilized onto a support via a covalent bond by using a support that has a functional group reactive with the amino group such as an aldehyde group or a carboxyl group on the surface. Such a functional group can be introduce onto a support according to a known method.

The oligonucleotide having an amino group being introduced can be immobilized onto a support which has been treated with glutaraldehyde or phenyl dithiocyanate. Treatment with phenyl dithiocyanate requires handling in a nonaqueous system. On the other hand, treatment with glutaraldehyde is convenient and preferable for the immobilization method of the present invention. If a slide glass treated with glutaraldehyde is used as a support, a solution containing an oligonucleotide having an amino group being introduced is first spotted onto the slide glass to form a Schiff base between the aldehyde group and the amine group. The slide glass is then subjected to reduction to convert the Schiff base into a stable amine. Thus, a slide glass onto which an oligonucleotide of interest is firmly immobilized can be prepared.

An oligonucleotide having modification other than the amino group can also be immobilized onto a support via a covalent bond according to a known procedure by selecting a support having a functional group suitable for the modification.

Detachment of an oligonucleotide immobilized onto a support via a covalent bond according to the immobilization method as described above during a washing or hybridization step is little. Therefore, it is useful for obtaining stable experimental results. Furthermore, an oligonucleotide immobilization rate in a dot area or a unit area can be increased by using the immobilization method. In other words, the density of an oligonucleotide immobilized in a unit area can be increased. Thus, the sensitivity of detecting a target nucleic acid can be increased as compared with conventional methods by using the support having oligonucleotides immobilized according to the above-mentioned method.

An immobilization rate of an oligonucleotide to a support can be evaluated by immobilizing an oligonucleotide labeled with a fluorescent substance (e.g., Rhodamine) onto a support, and measuring the intensity of fluorescence emitted from a dot on the support using a fluorescence image analyzer such as FMBIO II Multi-View (Takara Shuzo). The immobilization rate can be calculated according to equation 1 below.

Equation 1:

Immobilization rate (%) = 100 x (fluorescence intensity for

a dot on a support after immobilization and washing) /

(fluorescence intensity for a dot immediately after

immobilization)

The density of an immobilized oligonucleotide can be determined, for example, as follows. An oligonucleotide labeled with a fluorescent substance (e.g., Rhodamine) is immobilized. The intensity of fluorescence emitted from a dot in which the oligonucleotide is immobilized can be measured using a fluorescence image analyzer such as FMBIO II Multi-View. The amount of the oligonucleotide immobilized in the dot can be calculated on the basis of a calibration curve prepared for the amount of fluorescence-labeled oligonucleotide used and the fluorescence intensity.

The amount of an oligonucleotide immobilized in a unit area, that is, the density of an immobilized DNA, can be determined by measuring the area of the dot, for example, using the fluorescence image analyzer.

Alternatively, a predetermined amount of an oligonucleotide solution is spotted onto a support according to the immobilization method of the present invention, and the immobilization rate is determined. The amount of an immobilized oligonucleotide can be calculated based on the determined immobilization rate and the amount of the spotted oligonucleotide. The area of a formed dot can be determined, for example, by using a microscope. The density of the immobilized oligonucleotide can also be determined based on the amount of the immobilized oligonucleotide and the area determined as described above.

A material onto which an oligonucleotide is immobilized obtained as described above is also encompassed by the present invention.

The volume of an oligonucleotide solution spotted onto a support using GMS 417 Arrayer is about 50 to 100 picoliter (pl). An oligonucleotide is immobilized with an efficiency of 25% or more according to the method for immobilizing an oligonucleotide of the present invention as described in Examples below. Accordingly, if a solution containing an oligonucleotide at a concentration of 100 μM is spotted onto a support using the arrayer, a material onto which at least 1.25 femtomole (fmole) of the oligonucleotide is immobilized in a dot is prepared. A target nucleic acid can be detected with sufficient sensitivity by using such a material onto which an oligonucleotide is immobilized.

Furthermore, a material onto which a number of oligonucleotides are immobilized in a unit area on a support (e.g., a high-density oligonucleotide array having 36 or more dots formed in a area of 1 cm$^2$ on a support) can be prepared by using GMS 417 Arrayer. Such a high-density oligonucleotide array can be used to detect a target nucleic acid in a trace amount. Thus, it is useful for analyzing a gene in a limited amount of a sample. For example, a high-density oligonucleotide array having 100 dots or more, preferably 400 dots or more, more preferably 900 dots in a area of 1 cm$^2$ can be prepared using the above-mentioned instrument according to the method of the present invention.

A method of detecting a target nucleic acid using the material onto which an oligonucleotide is immobilized is also encompassed by the present invention. In this method, the material onto which an oligonucleotide is immobilized is hybridized with a target nucleic acid under stringent conditions.

As used herein, stringent conditions refer to those as described in Molecular cloning, A laboratory manual, 2nd ed., pp. 9.47-9.51 (1989, Cold Spring Harbor Laboratory). For example, a material onto which an oligonucleotide is immobilized is hybridized with a target nucleic acid under stringent conditions as follows, although the conditions may vary depending on, among others, the length of the base sequence in a portion of the target nucleic acid that hybridizes with the immobilized DNA. A Tm value is calculated based on the length of the portion to be hybridized. Hybridization is carried out at a temperature lower than the calculated Tm value by 20 to 25°C in an appropriate buffer such as a solution having a high ionic strength (e.g., 6 x SSC or 6 x SSPE). Usually, it is preferable to conduct washing at a temperature lower than the Tm value by 12 to 20°C while changing the salt concentrations of washing buffers in a subsequent washing step.

The target nucleic acid is not limited to specific one. For example, any nucleic acids can be used as a target to screen an oligonucleotide that hybridizes with the target nucleic acid among oligonucleotides immobilized onto a support. A target nucleic acid labeled by appropriate means, for example, by using a fluorescent substance, is usually used for efficient screening. A target nucleic acid being hybridized with an oligonucleotide on a support can be detected or quantified by conducting hybridization - washing steps as described above using a labeled target nucleic acid and then measuring the fluorescence on the support.

Furthermore, a DNA chip onto which a oligonucleotide is immobilized is useful for sequencing by hybridization (SBH) and analysis of single nucleotide polymorphism (SNP) of a gene.

As described above, by using the method for immobilizing an oligonucleotide onto a support of the present invention, the number of dots for oligonucleotides in a unit area on a support can be increased, the amount of an oligonucleotide immobilized in a dot can be increased, and detachment of an oligonucleotide during an experimental procedure can be reduced, without lowering the sensitivity of detecting a target nuclei acid. Furthermore, a target nucleic acid can be detected with a high efficiency and with high detection sensitivity by using the material onto which an oligonucleotide is immobilized provided according to the method of the present invention.

Examples

The following examples further illustrate the present invention in detail but are not to be construed to limit the scope thereof.

Solutions prepared so as to have the following compositions were diluted to given concentrations and used as buffers for various tests as described in Examples below.

20 x SSC: a solution of 0.3 M sodium citrate containing 3M sodium chloride. TE buffer: 10 mM tris-hydrochloride (pH 8.0) containing 1 mM EDTA. Phosphate buffered saline (PBS): prepared using a PBS tablet (Takara Shuzo). 500 mM carbonate buffer: prepared by mixing a 500 mM sodium carbonate solution and a 500 mM sodium hydrogencarbonate solution to adjust the pH to a given value. 500 mM morpholine buffer: prepared by adjusting the pH of 500 mM N-methylmorpholine to a given value with diluted hydrochloric acid. 50 mM borate buffer: prepared by adjusting the pH of a 50 mM boric acid solution containing 50 mM potassium chloride to a given value with 2 N sodium hydroxide.

Example 1

(1) Immobilization of oligonucleotide

An oligonucleotide which has a base sequence of SEQ ID NO:1 and to which Rhodamine X (PE Biosystems, hereinafter referred to as ROX) is attached at the 5'-terminus (hereinafter referred to as ROX oligo), and an oligonucleotide which also has a base sequence of SEQ ID NO:1 and to which an alkylamino linker (PE Biosystems) which has an alkyl chain of 6 carbons (C6) and Rhodamine X are attached at the 5'-terminus and at the 3'-termunus, respectively (hereinafter referred to as ROX amino oligo) were synthesized using a DNA synthesizer. These synthetic DNAs were dissolved in 50 mM N-methylmorpholine-hydrochloride (NMM) buffer (pH 9.5) or 50 mM carbonate buffer (pH 9.5) at a concentration of 10 μM to prepare oligonucleotide solutions.

On the other hand, immobilization was examined for an oligonucleotide dissolved in TE buffer as a control.

Aminoalkylsilane (AAS)-coated slide glasses (AAS-treated, Sigma) and AAS-coated slide glasses which were further soaked in a 2.5% glutaraldehyde (GA) aqueous solution for 1 hour, washed three times in distilled water and air-dried (AAS + GA-treated) were used.

0.2 μl each of the oligonucleotide solutions was spotted onto each of the slide glasses. After air-drying, the slide glasses were incubated at 37°C for 1 hour in a humidified incubator. The slide glasses were irradiated with ultraviolet at 60 mJ, and then soaked in a succinic acid solution for 15 minutes to block free amino groups. The succinic acid solution was prepared by dissolving 5 g of succinic anhydride (Nacalai Tesque) in 315 ml of N-methyl-2-pyridone (Nacalai Tesque) and further adding 35 ml of 0.2 M borate buffer (pH 8.0) thereto. Finally, the slide glasses were washed in a 0.2% SDS solution for 2 minutes followed by distilled water and dried at room temperature.

For the slide glass treated with glutaraldehyde, the washed slide glass was further soaked in a 3:1 mixture of phosphate buffered saline (PBS) - 100% ethanol containing 0.25% sodium boron hydride (Wako Pure Chemical Industries) for 5 minutes, and then washed and dried as described above in order to reduce the Schiff base formed between the aldehyde group on the support and the amino group in the DNA to a stable amine.

The immobilized oligonucleotides prepared as described above were subjected to stringent hybridization conditions as follows. 4 x SSC containing 0.2% SDS was spotted onto a region for spotting on each of the slide glasses. The spots were covered with cover glasses such that no air bubble was included. The sides were shut tightly with seals and the slide glasses were incubated at 37 to 40°C for 2 to 6 hours. After removing the seals, the slide glasses were washed in 0.2 x SSC containing 0.1% SDS for 30 minutes followed by 0.2 x SSC for 30 minutes and dried.

(2) Quantification of immobilized oligonucleotide

Fluorescence intensities were measured immediately after immobilization and after treatment under hybridization conditions using a fluorescence image analyzer FMBIO II Multi-View (Takara Shuzo). Immobilization rates were calculated based on the values of the respective fluorescence intensities. The results are shown in Table 1.

Table 1

| Solvent for oligonucleotide | Type of oligonucleotide | Surface treatment of slide glass | Immobilization rate (%) |
|---|---|---|---|
| TE buffer | ROX oligo | AAS | 0.7 |
| | | AAS + GA | 1.4 |
| | ROX amino oligo | AAS | 0.9 |
| | | AAS + GA | 1.6 |
| NMM buffer | ROX oligo | AAS | 5.8 |
| | | AAS + GA | 13.2 |
| | ROX amino oligo | AAS | 19.1 |
| | | AAS + GA | 65.6 |
| Carbonate buffer | ROX oligo | AAS | 4.4 |
| | | AAS + GA | 7.7 |
| | ROX amino oligo | AAS | 16.6 |
| | | AAS + GA | 25.7 |

As shown in Table 1, use of N-methylmorpholine-hydrochloride (NMM) buffer or carbonate buffer was shown to increase the immobilization rate for the oligonucleotide having an amino group at the 5'-terminus (ROX amino oligo). The immobilization rate was further increased when the AAS slide glass treated with glutaraldehyde (AAS + GA-treated) was used. On the other hand, the immobilization rate for the control in which TE buffer was used was low regardless of the type the immobilized oligonucleotide or the surface treatment of the support.

Example 2

(1) Immobilization of oligonucleotide

An oligonucleotide TFR AS7 having a base sequence of SEQ ID NO:2 was synthesized on the basis of the base sequence for human transferrin receptor (TFR) gene. An amino groups was added at the 5'-terminus of the oligonucleotide using a C12 alkylamino linker (PE Biosystems). Two oligonucleotide derivatives were prepared from this oligonucleotide by further adding an ROX label at the 3'-terminus (TFR AS7 3-ROX) or replacing the amino group at the 5'-terminus by ROX (without amino group, TFR AS7 5-ROX). Each of the oligonucleotides was dissolved in 50 mM carbonate buffer (pH 9.5), 3 x SSC or TE buffer at a concentration of 1 or 10 μM.

AAS-coated slide glasses were treated with glutaraldehyde for activation as described in Example 1. 0.2 μl each of the solutions was spotted onto each of the slide glasses and immobilized as described in Example 1. Immobilization was carried out in two ways, i.e., with ("with reduction" in Table 2) or without ("without reduction" in Table 2) the reduction using sodium boron hydride. After immobilization, the slide glasses were washed in water at 25°C or 95°C.

(2) Hybridization and signal detection

A signal from ROX immobilized on each of the slide glasses prepared in (1) above was analyzed by scanning using FMBIO II Multi-View. The results obtained using carbonate buffer, 3 x SSC or TE buffer are shown in Table 2. In the table, - arid + represents that no signal was observed and that a signal was observed, respectively. + to 4+ represent the intensities of the signals. Increase in the number indicates increase in the signal intensity.

a. Buffer

Oligonucleotide immobilization rates for the buffers were determined as follows: carbonate buffer > 3 x SSC > TE buffer. Among these, when the solution containing the oligonucleotide at a concentration of 1 μM was spotted using TE buffer, no signal was observed, indicating that this buffer is not suitable for immobilizing an oligonucleotide.

b. Oligonucleotide

It was demonstrated that a higher immobilization efficiency is generally accomplished by using an oligonucleotide having an amino group at the 5'-terminus (TFR AS7 3-ROX) as compared with an oligonucleotide without an amino group (TFR AS7 5-ROX). In particular, when the immobilization was carried out with the reduction, no difference in the signals for the two washing temperatures (25°C and 95°C) was observed regardless of the buffer used (carbonate buffer or 3 x SSC), indicating that the oligonucleotide is stably immobilized.

As described above, it was demonstrated that reduction using sodium boron hydride is effective and that immobilization using carbonate buffer yields good results when an oligonucleotide is immobilized onto an AAS-coated slide glass treated with glutaraldehyde.

Table 2

| Carbonate buffer | | | | |
|---|---|---|---|---|
| Slide glass | Oligonucleotide | Washing temperature (°C) | Oligonucleotide concentration | |
| | | | 1 µM | 10 µM |
| AAS-coated slide glass treated with glutaraldehyde (with reduction) | TFR AS7 3-ROX | 25 | 3+ | 4+ |
| | | 95 | 3+ | 4+ |
| | TFR AS7 5-ROX | 25 | + | 2+ |
| | | 95 | + | 2+ |
| AAS-coated slide glass treated with glutaraldehyde (without reduction) | TFR AS7 3-ROX | 25 | 2+ | 3+ |
| | | 95 | + | 2+ |
| | TFR AS7 5-ROX | 25 | + | 2+ |
| | | 95 | + | + |
| 3 x SSC | | | | |
| Slide glass | Oligonucleotide | Washing temperature (°C) | Oligonucleotide concentration | |
| | | | 1 µM | 10 µM |
| AAS-coated slide glass treated with glutaraldehyde (with reduction) | TFR AS7 3-ROX | 25 | + | 2+ |
| | | 95 | + | 2+ |
| | TFR AS7 5-ROX | 25 | - | + |
| | | 95 | - | + |
| AAS-coated slide glass treated with glutaraldehyde (without reduction) | TFR AS7 3-ROX | 25 | + | 2+ |
| | | 95 | - | + |
| | TFR AS7 5-ROX | 25 | - | + |
| | | 95 | - | + |
| TE buffer | | | | |
| Slide glass | Oligonucleotide | Washing temperature (°C) | Oligonucleotide concentration | |
| | | | 1 µM | 10 µM |
| AAS-coated slide glass treated with glutaraldehyde (with reduction) | TFR AS7 3-ROX | 25 | - | + |
| | | 95 | - | - |
| | TFR AS7 5-ROX | 25 | - | + |
| | | 95 | - | - |

Table 2   (continued)

| TE buffer | | | | |
|---|---|---|---|---|
| Slide glass | Oligonucleotide | Washing temperature (°C) | Oligonucleotide concentration | |
| | | | 1 μM | 10 μM |
| AAS-coated slide glass treated with glutaraldehyde (without reduction) | TFR AS7 3-ROX | 25 | - | + |
| | | 95 | - | + |
| | TFR AS7 5-ROX | 25 | - | + |
| | | 95 | - | - |

Example 3

(1) Immobilization of oligonucleotide

Aminopropyltriethoxysilane (APS)-coated slide glasses (Matsunami Glass) were treated with glutaraldehyde for activation as described in Example 1. Eight oligonucleotides, c-K-ras/61Q (wild type), c-K-ras/61K, c-K-ras/61E, c-K-ras/61R, c-K-ras/61P, c-K-ras/61L, c-K-ras/61H1 and c-K-ras/61H2 were synthesized. These oligonucleotides correspond to base sequences of wild type K-ras gene as well as mutant K-ras genes each encoding a protein in which the amino acid at position 61 of wild type K-ras protein, Gln, is replaced by Lys, Glu, Arg, Pro, Leu or His (two types). The base sequences of the synthesized eight oligonucleotides are shown in SEQ ID NOS:3 to 10. In each of SEQ ID NOS:4 to 10, the mutated codon is located at positions 10 to 12. The oligonucleotide TFR AS7 (SEQ ID NO:2) synthesized in Example 2 was used as a negative control. Amino groups were added to the 5'-termini of these oligonucleotides through C12 alkyl chains. Each of the oligonucleotides was dissolved in 50 mM carbonate buffer (pH 9.5) at a concentration of 1 mM, 100 μM, 10 μM or 1 μM (corresponding to 6.6, 0.66, 0.066 or 0.0066 mg/ml, respectively). 0.2 μl each of the solutions containing one of the oligonucleotides at the respective concentrations was spotted onto each of the slide glasses. The slide glasses were subjected to immobilization, ultraviolet irradiation, blocking and reduction using sodium boron hydride as described in Example 1.

(2) Hybridization and signal detection

PCRs were carried out using eight template DNAs contained in ras Mutant Set c-Ki-ras codon 61 (Takara Shuzo) as templates and ras Gene Primer Set c-Ki-ras/61 (Takara Shuzo). The amplified 128-bp PCR products were purified using CentriSep column (PE Biosystems) and recovered. Reverse primers were used after labeling with ROX. The thus obtained ROX-labeled PCR products were used as probes.
The concentrations of the eight probes were adjusted to 3 μM with a hybridization solution (4 x SSC containing 0.2% SDS). The resulting solutions were spotted onto the slide glasses onto which oligonucleotides had been immobilized prepared as described in Example 2-(1), and covered with cover glasses. After incubation at 50°C for 2 hours in a humidified incubator, the cover glasses were removed. The slide glasses were washed at 25°C in 0.2 x SSC containing 0.1% SDS for 30 minutes followed by 0.2 x SSC for 30 minutes.
After drying the slide glasses, fluorescence from ROX on each of the slide glasses was detected using FMBIO II Multi-View to determine the amount of the probe hybridized with each of the oligonucleotides. The results obtained for three probes, c-K-ras codon 61 AAA, c-K-ras codon 61 GAA and c-K-ras codon 61 CGA (corresponding to the oligonucleotides c-K-ras/61K, c-K-ras/61E and c-K-ras/61R, respectively), are shown in Table 3. In the table, - and + represents that no signal was observed and that a signal was observed, respectively. + to 6+ represent the intensities of the signals. Increase in the number indicates increase in the signal intensity.
As shown in Table 3, for each probe, an intense signal was observed for a dot for an oligonucleotides in which 100% of the sequence matched with that of the corresponding probe used. On the other hand, only a weak signal was observed for a dot for a mismatched oligonucleotide. Thus, it was demonstrated the material to which an oligonucleotide is immobilized prepared as described above can be used to distinguish base sequences of target nucleic acids (probes). A more intense signal was observed when the concentration of the oligonucleotide to be spotted was higher.

Table 3

| Probe | Oligonucleotide | Oligonucleotide concentration (μM) | | |
|---|---|---|---|---|
| | | 10 | 100 | 1000 |
| c-K-ras codon 61 AAA (corresponding to c-K-ras/61K) | c-K-ras/61Q | + | 2+ | 3+ |
| | c-K-ras/61K | 2+ | 3+ | 5+ |
| | c-K-ras/61E | + | 2+ | 3+ |
| | c-K-ras/61R | - | - | - |
| | c-K-ras/61P | - | - | - |
| | c-K-ras/61L | - | - | - |
| | c-K-ras/61H1 | - | - | - |
| | c-K-ras/61H2 | - | - | - |
| | TFR AS7 | - | - | - |
| c-K-ras codon 61 GAA (corresponding to c-K-ras/61E) | c-K-ras/61Q | + | + | + |
| | c-K-ras/61K | + | + | + |
| | c-K-ras/61E | 3+ | 5+ | 6+ |
| | c-K-ras/61R | - | - | - |
| | c-K-ras/61P | - | - | - |
| | c-K-ras/61L | - | - | - |
| | c-K-ras/61H1 | - | - | - |
| | c-K-ras/61H2 | - | - | - |
| | TFR AS7 | - | - | - |
| c-K-ras codon 61 CGA (corresponding to c-K-ras/61R) | c-K-ras/61Q | - | - | + |
| | c-K-ras/61K | - | - | - |
| | c-K-ras/61E | - | - | - |
| | c-K-ras/61R | 4+ | 5+ | 6+ |
| | c-K-ras/61P | - | - | - |
| | c-K-ras/61L | - | - | - |
| | c-K-ras/61H1 | - | - | - |
| | c-K-ras/61H2 | - | - | - |
| | TFR AS7 | - | - | - |

Example 4

(1) Immobilization of oligonucleotide

APS-coated slide glasses and APS-coated slide glasses treated with glutaraldehyde for activation as described in Example 1 were prepared. 0.2 μl each of solutions each containing one of the oligonucleotides c-Ki-ras/61Q, c-Ki-ras/61K or TFR AS7 (a negative control) used in Example 3 at a concentration of 1 μM to 1 mM was spotted onto the two types of slide glasses, and immobilized as described in Example 1. Reduction using sodium boron hydride was omitted for the APS-coated slide glasses without the glutaraldehyde treatment.

(2) Hybridization and signal detection

A PCR was carried out for c-Ki-ras codon 61 AAA (corresponding to the oligonucleotide c-K-ras/61K) in ras Mutant Set c-Ki-ras codon 61 as described in Example 2 to prepare an ROX-labeled DNA probe. The concentration of the probe was adjusted to 2 μM with a hybridization solution (4 x SSC containing 0.2% SDS). The resulting solution was spotted onto each of the above-mentioned slide glasses onto which the oligonucleotides had been immobilized, and covered with a cover glass. After incubation at 37°C for 2 hours or 50°C for 2 hours in a humidified incubator, the cover glasses were removed. The slide glasses were washed at 25°C in 0.2 x SSC containing 0.1% SDS for 30 minutes followed by 0.2 x SSC for 30 minutes. After drying the slide glasses, a signal on each of the slide glasses was analyzed by scanning using FMBIO II Multi-View. The results are shown in Table 4. In the table, - and + represents that no signal was observed and that a signal was observed, respectively. + to 5+ represent the intensities of the signals. Increase in the number indicates increase in the signal intensity.

For the APS slide glasses treated with glutaraldehyde, a specific signal was observed for the oligonucleotide in which 100% of the sequence matched with that of the probe regardless of the concentration of the oligonucleotide used for immobilization or the hybridization temperature (c-k-ras/61K). Although the signal intensity depended on the concentration of the oligonucleotide used, a sufficiently intense signal could be observed and a matched probe could be distinguished from a mismatched probe even if a solution containing an oligonucleotide at a concentration of 1 μM was spotted onto a dot. The difference between a signal for a matched probe and a signal for a mismatched probe observed using a slide glass without glutaraldehyde treatment was smaller than that observed using a slide glass treated with glutaraldehyde. These results shows that a material onto which an oligonucleotide is immobilized which has a high specificity to a base sequence can be obtained by immobilizing an oligonucleotide onto a slide glass treated with glutaraldehyde.

Table 4

| APS-coated slide glass | | | | | |
|---|---|---|---|---|---|
| Hybridization temperature | Oligonucleotide | Oligonucleotide concentration (μM) | | | |
| | | 1 | 10 | 100 | 1000 |
| 37°C | c-K-ras/61Q | - | + | + | 2+ |
| | c-K-ras/61K | 2+ | 2+ | 4+ | 4+ |
| | TFR AS7 | - | - | - | - |
| 50°C | c-K-ras/61Q | + | + | 2+ | 2+ |
| | c-K-ras/61K | 2+ | 2+ | 4+ | 4+ |
| | TFR AS7 | - | - | - | - |
| APS-coated slide glass treated with glutaraldehyde | | | | | |
| Hybridization temperature | Oligonucleotide | Oligonucleotide concentration (μM) | | | |
| | | 1 | 10 | 100 | 1000 |
| 37°C | c-K-ras/61Q | + | + | 2+ | 2+ |
| | c-K-ras/61K | 2+ | 3+ | 4+ | 5+ |
| | TFR AS7 | - | - | - | - |
| 50°C | c-K-ras/61Q | - | - | + | + |
| | c-K-ras/61K | 2+ | 3+ | 4+ | 5+ |
| | TFR AS7 | - | - | - | - |

Example 5

(1) Immobilization of oligonucleotide

Oligonucleotides c-Ki-ras/61Q and TFR AS7 (negative control) as described in Example 3 each having an amino group at the end of a C3, C6 or C12 alkyl chain being attached to the 5'-terminus of the oligonucleotide were synthesized. Alkylamino linkers from PE Biosystems were used for the synthesis of these oligonucleotides. Each of the oligonucleotides was dissolved in 50 mM carbonate buffer (pH 9.5) at a concentration 1, 10 or 100 μM. 0.2 μl each of the solutions was spotted onto an APS-coated slide glass or an APS-coated slide glass treated with glutaraldehyde, and immobilized as described in Example 1. Reduction using sodium boron hydride was omitted for the APS-coated slide glasses without the glutaraldehyde treatment.

(2) Hybridization and signal detection

The probe used in Example 3 (c-K-ras codon 61 CAA; corresponding to the oligonucleotide c-K-ras/61Q) was dissolved in a hybridization solution (4 x SSC containing 0.2% SDS) at a concentration of 2 or 4 μM. The resulting solutions were spotted onto each of the above-mentioned slide glasses, and covered with cover glasses. After incubation at 37°C for 2 hours in a humidified incubator, the cover glasses were removed. The slide glasses were washed at 25°C in 0.2 x SSC containing 0.1% SDS for 30 minutes followed by 0.2 x SSC for 30 minutes. After drying the slide glasses, the signals on the slide glasses were analyzed by scanning using FMBIO II Multi-View. The results are shown in Table 5. In the table, - and + represents that no signal was observed and that a signal was observed, respectively. + to 4+ represent the intensities of the signals. Increase in the number indicates increase in the signal intensity.
When the glutaraldehyde-treated slide glass was used, a more intense signal was observed by using a longer alkyl chain (spacer) between the terminus of the oligonucleotide and the amino group (C3 < C6 < C12). Among oligonucleotides having the same alkyl chain in length, the signal intensity varied depending on the concentration of the oligonucleotide in the solution used for immobilization. On the other hand, when the APS-coated slide glass was used, no obvious correlation was observed between the length of the alkyl chain and the signal intensity. Furthermore, it was demonstrated that a more intense signal is observed using a slide glass treated with glutaraldehyde provided that the same oligonucleotide and the same buffer are used.

Table 5

| APS-coated slide glass | | | | | |
|---|---|---|---|---|---|
| Probe (concentration) | Oligonucleotide | | Oligonucleotide concentration (μM) | | |
| | | | 1 | 10 | 100 |
| c-K-ras codon 61 CAA (4 μM) | c-K-ras /61Q | C3 | + | + | + |
| | | C6 | + | + | 2+ |
| | | C12 | + | + | 2+ |
| | TFR AS7 | C3 | - | - | - |
| | | C6 | - | - | - |
| | | C12 | - | - | - |
| c-K-ras codon 61 CAA (2 μM) | c-K-ras /61Q | C3 | + | + | + |
| | | C6 | + | + | 2+ |
| | | C12 | + | + | 2+ |
| | TFR AS7 | C3 | - | - | - |
| | | C6 | - | - | - |
| | | C12 | - | - | - |
| APS-coated slide glass treated with glutaraldehyde | | | | | |
| Probe (concentration) | Oligonucleotide | | Oligonucleotide concentration (μM) | | |
| | | | 1 | 10 | 100 |
| c-K-ras codon 61 CAA (4 μM) | c-K-ras /61Q | C3 | - | + | + |
| | | C6 | + | + | 2+ |
| | | C12 | + | 3+ | 4+ |
| | TFR AS7 | C3 | - | - | - |
| | | C6 | - | - | - |
| | | C12 | - | - | - |

Table 5   (continued)

| APS-coated slide glass treated with glutaraldehyde | | | | | |
| Probe (concentration) | Oligonucleotide | | Oligonucleotide concentration (μM) | | |
| | | | 1 | 10 | 100 |
| c-K-ras codon 61 CAA (2 μM) | c-K-ras /61Q | C3 | + | + | + |
| | | C6 | + | + | 2+ |
| | | C12 | + | 2+ | 3+ |
| | TFR AS7 | C3 | - | - | - |
| | | C6 | - | - | - |
| | | C12 | - | - | - |

Example 6

(1) Preparation of microarray

Solutions each containing one of the eight K-ras 61 oligonucleotides and the oligonucleotide TFR AS7 as a negative control used in Example 3 at a concentration of 1 mM or 100 μM in carbonate buffer were spotted onto AAS-coated slide glasses treated with glutaraldehyde using GMS 417 Arrayer (Genetic MicroSystems). The slide glasses were subjected to immobilization as described in Example 1.

(2) Hybridization an signal detection

Hybridization was carried out using the eight ROX-labeled probes prepared as described in Example 3. The probes were dissolved in a hybridization solution (4 x SSC containing 0.2% SDS) at a concentration of 3 μM. The resulting solutions were spotted onto the above-mentioned slide glasses, and covered with cover glasses. After incubation at 37°C for 4 hours in a humidified incubator, the cover glasses were removed. The slide glasses were washed at 25°C in 0.2 x SSC containing 0.1% SDS for 30 minutes followed by 0.2 x SSC for 30 minutes. After drying the slide glasses, a signal on each of the slide glasses was analyzed by scanning using GMS 418 Array Scanner (Genetic MicroSystems). The results obtained using c-K-ras codon 61 GAA, c-K-ras codon 61 CGA and c-K-ras codon 61 CAT (corresponding to the oligonucleotides c-K-ras/61E, c-K-ras/61R and c-K-ras/61H1, respectively) as probes are shown in Table 6. In the table, - and + represents that no signal was observed and that a signal was observed, respectively. + to 7+ represent the intensities of the signals. Increase in the number indicates increase in the signal intensity.

For each probe, an intense signal was observed for a dot for an oligonucleotide in which 100% of the sequence matched with that of the corresponding probe used. On the other hand, only a weak signal was observed for a dot for a mismatched oligonucleotide. Although a more intense signal was observed when the concentration of the spotted oligonucleotide was higher, a base sequence matched with that of the probe could be distinguished from a mismatched base sequence even if a solution containing an oligonucleotide at a concentration of 100 μM was spotted onto a dot.

Table 6

| Probe | Oligonucleotide | Oligonucleotide concentration | |
|---|---|---|---|
| | | 100 μM | 1 mM |
| c-K-ras codon 61 GAA (corresponding to c-K-ras/61E) | c-K-ras/61Q | 3+ | 4+ |
| | c-K-ras/61K | 3+ | 4+ |
| | c-K-ras/61E | 5+ | 7+ |
| | c-K-ras/61R | 2+ | 3+ |
| | c-K-ras/61P | 2+ | 3+ |
| | c-K-ras/61L | + | 2+ |
| | c-K-ras/61H1 | + | 2+ |
| | c-K-ras/61H2 | + | 2+ |
| | TFR AS7 | - | - |
| c-K-ras codon 61 CGA (corresponding to c-K-ras/61R) | c-K-ras/61Q | 2+ | 3+ |
| | c-K-ras/61K | 2+ | 2+ |
| | c-K-ras/61E | 2+ | 2+ |
| | c-K-ras/61R | 5+ | 7+ |
| | c-K-ras/61P | 3+ | 4+ |
| | c-K-ras/61L | 3+ | 4+ |
| | c-K-ras/61H1 | 2+ | 3+ |
| | c-K-ras/61H2 | 2+ | 3+ |
| | TFR AS7 | - | - |
| c-K-ras codon 61 CAT (corresponding to c-K-ras/61H1) | c-K-ras/61Q | 2+ | 3+ |
| | c-K-ras/61K | 2+ | 3+ |
| | c-K-ras/61E | 2+ | 3+ |
| | c-K-ras/61R | 2+ | 3+ |
| | c-K-ras/61P | 2+ | 3+ |
| | c-K-ras/61L | 2+ | 3+ |
| | c-K-ras/61H1 | 5+ | 7+ |
| | c-K-ras/61H2 | 3+ | 4+ |
| | TFR AS7 | - | - |

Industrial Applicability

A method for immobilizing an oligonucleotide onto a solid support which is useful for the preparation of a DNA microarray and the like is provided. A material onto which an oligonucleotide is immobilized according to said method and a method for detecting a target nucleic acid using said material are also provided.

Sequence Listing Free Text

SEQ ID NO: 1: Designed oligonucleotide for testing immobilization efficiency.
SEQ ID NO: 2: Designed oligonucleotide designated as TFR AS7 corresponding to a portion of human transferrin receptor (TFR) gene.

SEQ ID NO: 3: Designed oligonucleotide designated as c-K-ras/61Q corresponding to a portion of wild type K-ras gene.

SEQ ID NO: 4: Designed oligonucleotide designated as c-K-ras/61K corresponding to a portion of mutant K-ras gene.

SEQ ID NO: 5: Designed oligonucleotide designated as c-K-ras/61E corresponding to a portion of mutant K-ras gene.

SEQ ID NO: 6: Designed oligonucleotide designated as c-K-ras/61R corresponding to a portion of mutant K-ras gene.

SEQ ID NO: 7: Designed oligonucleotide designated as c-K-ras/61P corresponding to a portion of mutant K-ras gene.

SEQ ID NO: 8: Designed oligonucleotide designated as c-K-ras/61L corresponding to a portion of mutant K-ras gene.

SEQ ID NO: 9: Designed oligonucleotide designated as c-K-ras/61H1 corresponding to a portion of mutant K-ras gene.

SEQ ID NO:10: Designed oligonucleotide designated as c-K-ras/61H2 corresponding to a portion of mutant K-ras gene.

## Claims

1. A method for immobilizing an oligonucleotide onto a support, the method comprising spotting a buffer containing an oligonucleotide onto a support, and immobilizing the oligonucleotide onto the support via a covalent bond.

2. The method according to claim 1, wherein a functional group is introduced into the oligonucleotide.

3. The method according to claim 2, wherein the functional groups is introduced at a terminus of the oligonucleotide.

4. The method according to claim 2 or 3, wherein an amino group is introduced into the oligonucleotide.

5. The method according to any one of claims 1 to 4, wherein the support has a functional group.

6. The method according to claim 5, wherein the support has an aldehyde group.

7. The method according to any one of claims 1 to 6, wherein the oligonucleotide is immobilized onto the support through a spacer between the oligonucleotide and the surface of the support.

8. The method according to any one of claims 1 to 7, wherein 200 nl or less of the buffer containing the oligonucleotide at a concentration of 1 $\mu$M or more is spotted onto the support.

9. The method according to any one of claims 1 to 8, wherein the support is made from glass or quartz, or is a material prepared by treating the surface of glass or quartz.

10. The method according to any one of claims 1 to 9, wherein the buffer contains at least one substance selected from the group consisting of morpholine, a morpholine derivative, a salt thereof and a carbonate.

11. The method according to claim 10, wherein the concentration of at least one substance selected from the group consisting of morpholine, a morpholine derivative, a salt thereof and a carbonate in the buffer is 10 to 500 mM.

12. A material onto which an oligonucleotide is immobilized, which is prepared according to the method defined by any one of claims 1 to 11.

13. The material according to claim 12, wherein the oligonucleotide is immobilized at 1.25 fmole/dot or more.

14. A method for detecting a target nucleic acid, the method comprising detecting a target nucleic acid by using the material onto which an oligonucleotide is immobilized defined by claim 12 or 13.

15. The method according to claim 14, comprising hybridizing the material onto which an oligonucleotide is immobilized with the target nucleic acid under stringent conditions.

## INTERNATIONAL SEARCH REPORT

| | International application No. |
|---|---|
| | PCT/JP99/06867 |

| A. CLASSIFICATION OF SUBJECT MATTER . |
|---|
| Int.Cl⁷ C12N15/00, C12Q1/68, G01N33/50 |
| |
| According to International Patent Classification (IPC) or to both national classification and IPC |

| B. FIELDS SEARCHED |
|---|
| Minimum documentation searched (classification system followed by classification symbols) |
| Int.Cl⁷ C12N15/00, C12Q1/68, G01N33/50 |
| |
| Documentation searched other than minimum documentation to the extent that such documents are included in the fields searched |
| |
| Electronic data base consulted during the international search (name of data base and, where practicable, search terms used) |
| WPI(DIALOG),BIOSIS(DIALOG) |

| C. DOCUMENTS CONSIDERED TO BE RELEVANT |

| Category* | Citation of document, with indication, where appropriate, of the relevant passages | Relevant to claim No. |
|---|---|---|
| X | Zhen Guo et al. "Direct fluorescence analysis of genetic polymorphisms by hybridization with oligonucleotide arrays on glass supports" Nucleic Asids Research (1994) Vol.22, No.24, P.5456-5465 | 1-15 |
| X | WO, 89/11548, A (Cetus Corporation), 30 November, 1989 (30.11.89) & EP, 451141, A & JP, 3-504328, A | 1-15 |
| PX | EP, 895082, A (Canon Inc.), 03 February, 1999 (03.02.99) & JP, 11-187900, A | 1-15 |

| ☐ | Further documents are listed in the continuation of Box C. | ☐ | See patent family annex. |
|---|---|---|---|

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance | |
| "E" earlier document but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| 09 February, 2000 (09.02.00) | 15 February, 2000 (15.02.00) |

| Name and mailing address of the ISA/ | Authorized officer |
|---|---|
| Japanese Patent Office | |
| Facsimile No. | Telephone No. |

Form PCT/ISA/210 (second sheet) (July 1992)